**Europäisches Patentamt**

**European Patent Office**

**Office européen des brevets**

(19)

(11) Veröffentlichungsnummer: **0 079 497**
**B1**

(12)

# EUROPÄISCHE PATENTSCHRIFT

(45) Veröffentlichungstag der Patentschrift:
**10.04.85**

(21) Anmeldenummer: **82109916.5**

(22) Anmeldetag: **27.10.82**

(51) Int. Cl.⁴: **C 07 C 51/14**, C 07 C 53/124, C 07 C 67/38, C 07 C 69/24, B 01 D 53/34

(54) **Verfahren zur Herstellung von Isobuttersäure oder ihren Alkylestern.**

(30) Priorität: **14.11.81 DE 3145311**

(43) Veröffentlichungstag der Anmeldung:
**25.05.83 Patentblatt 83/21**

(45) Bekanntmachung des Hinweises auf die Patenterteilung:
**10.04.85 Patentblatt 85/15**

(84) Benannte Vertragsstaaten:
**DE FR GB IT NL**

(56) Entgegenhaltungen:
**BE - A - 888 902**
**DE - A - 1 567 494**
**DE - A - 2 649 188**

**CHEMICAL ABSTRACTS, Band 83, Nr. 20, 17. November 1975, Nr. 168149t, Columbus, Ohio, USA**

(73) Patentinhaber: **Röhm GmbH, Kirschenallee Postfach 4242, D-6100 Darmstadt 1 (DE)**

(72) Erfinder: **Besecke, Siegmund, Dr. Dipl.-Chem., Auf dem Kreuzberg 6, D-6104 Seeheim-Jugenheim (DE)**
Erfinder: **Siegert, Hermann-Josef, Dr. Dipl.-Chem., Inselstrasse 21, D-6100 Darmstadt (DE)**
Erfinder: **Schröder, Günter, Dr. Dipl.-Chem., Leipziger Strasse 7, D-6105 Ober-Ramstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Beschreibung

*Technisches Gebiet*

Die Erfindung betrifft das im Oberbegriff des Patentanspruches bezeichnete Verfahren, insbesondere seine Durchführung im technischen Massstab. Während die grundsätzliche Verfahrensweise zur Herstellung von Isobuttersäure oder ihren Estern durch Kochsche Synthese aus verschiedenen Veröffentlichungen bekannt ist, sind die Probleme, die sich aus der Aufarbeitung von Nebenbestandteilen des Reaktionsgemisches ergeben, bisher nicht behandelt worden. Die Erfindung betrifft das Problem der Ausschleusung von Nebenbestandteilen aus dem gasförmigen Anteil des Reaktionsgemisches.

Die gasförmigen Ausgangsstoffe der Kochschen Synthese, nämlich Propylen und Kohlenmonoxid, enthalten stets kleine Mengen von Verunreinigungen, wie z.B. Kohlendioxid, Wasserstoff, Methan und andere gesättigte Kohlenwasserstoffe, Stickstoff usw. Zusätzlich können bei der Umsetzung gasförmige Nebenprodukte entstehen, die nur zum Teil oder gegebenenfalls gar nicht weiterreagieren, beispielsweise Isopropylfluorid. Da das Reaktionsprodukt Isobuttersäure oder Isobuttersäureester in der flüssigen Phase des Reaktionsgemisches entsteht und aus dieser gewonnen wird, braucht die gasförmige Phase des Reaktionsgemisches im Prinzip nicht ausgetauscht zu werden, wenn man die bei der Umsetzung verbrauchten Anteile laufend ersetzt. Dabei würden sich die erwähnten Nebenbestandteile allmählich anreichern. Es ist üblich, solche Bestandteile nach einer gewissen Anreicherung bei einem kontinuierlichen Verfahren dadurch auszuschleusen, dass man einen Teil der Gasphase laufend abnimmt, worin der Anteil der Nebenbestandteile so gross ist, dass er der laufenden Zuführung und Neubildung entspricht. Der abgenommene Teil der Gasphase würde jedoch erhebliche Mengen Fluorwasserstoff enthalten, der nicht in die Atmosphäre gelangen darf. Man müsste ihn daher, beispielsweise mit Kalkmilch, aus dem Abgas extrahieren. Diese Problemlösung ist wegen des Verlustes an Fluorwasserstoff unbefriedigend.

*Stand der Technik*

Eine sehr eingehende Untersuchung der Kochschen Synthese von Isobuttersäure unter Verwendung von Fluorwasserstoff als Kochschem Katalysator stammt von Y. Takezaki (,,Bulletin of The Japan Petroleum Institute", vol. 8, 1966, 31 bis 38). Das Problem der Beseitigung von Nebenbestandteilen des Reaktionsgemisches wird dort nicht behandelt. Das gleiche trifft für andere Beschreibungen dieses Verfahrens in US-A Nrn. 2975199 und 3052698 und EP-A Nr. 31886 zu. Die letztgenannte Druckschrift erwähnt zwar die Rückführung der niedrigsiedenden Bestandteile des Reaktionsgemisches, vor allem Fluorwasserstoff und Propylen, in den Reaktor, ebenso die Zerlegung von Nebenprodukten, wie Isobuttersäurefluorid, jedoch ist die Ausschleusung von Bestandteilen der Gasphase nicht vorgesehen. Der dem Reaktionsprodukt noch anhaftende Fluorwasserstoff soll durch Adsorption an Bauxit gebunden werden, jedoch können auf diese Weise nur Spuren von Fluorwasserstoff entfernt werden.

*Aufgabe*

Durch die Erfindung sollte die Kochsche Synthese von Isobuttersäure oder ihren Alkylestern bei dem im Oberbegriff des Patentanspruches beschriebenen Verfahren durch die Ausschleusung von Nebenbestandteilen des gasförmigen Anteils des Reaktionsgemisches in der Weise verbessert werden, dass dabei kein Fluorwasserstoff in die Atmosphäre gelangt oder als unbrauchbares Umsetzungsprodukt verloren geht oder unter Energie- oder Materialeinsatz aufgearbeitet werden muss.

*Lösung und vorteilhafte Wirkungen*

Die gestellte Aufgabe wird durch das im kennzeichnenden Teil des Patentanspruches angegebene Verfahren gelöst. Da für die Umsetzung ohnehin Wasser oder ein niederes Alkanol benötigt wird, bedeutet der Einsatz dieses Wassers oder Alkanols zur Rückgewinnung des Fluorwasserstoffs aus dem abgetrennten Teil der Gasphase keinen zusätzlichen Materialaufwand. Der Fluorwasserstoff wird aus dem Abgasstrom so weitgehend herausgewaschen, dass die im Abgas verbleibenden Restmengen vernachlässigbar klein sind und erforderlichenfalls an einer nachgeschalteten Absorptionssäule an einem basischen Absorptionsmittel, wie z.B. Bauxit, Kalk oder Kalkmilch, festgehalten werden können. Das Wasser oder Alkanol mit dem aufgenommenen Fluorwasserstoff kann unmittelbar in das Reaktionsgemisch eingeleitet werden, ohne dass es einer vorherigen Zerlegung, Konzentrierung oder sonstigen Aufbereitung bedarf. Eine Belastung der Abluft oder des Abwassers der Fabrikationsanlage mit Fluorwasserstoff wird mit Sicherheit vermieden.

*Gewerbliche Anwendbarkeit*

Es gibt verschiedene Ausführungsformen der Kochschen Synthese. Die klassische Kochsche Synthese wird zweistufig ausgeführt; dabei wird in der ersten Stufe in Gegenwart des Kochschen Katalysators ein Olefin mit Kohlenmonoxid umgesetzt. Das entstehende Zwischenprodukt wird in einer zweiten Stufe mit Wasser oder einem Alkanol zur Carbonsäure oder ihrem Ester weiter umgesetzt. Die beiden Verfahrensstufen werden in getrennten Reaktionsräumen durchgeführt. Bei einer einstufigen Verfahrensweise wird dem Reaktionsgemisch von vornherein Wasser oder ein Alkanol zugesetzt, so dass die Carbonsäure oder ihr Ester unmittelbar entstehen. Die Erfindung kann bei beiden Verfahrensvarianten angewendet werden, indem man das Fluorwasserstoff enthaltende Wasser oder Alkanol in den entsprechenden Reaktionsraum einführt.

Mit besonderem Vorteil wird die Erfindung bei der Herstellung der freien Isobuttersäure angewandt, wobei das zur Umsetzung eingesetzte Wasser als Absorptionsmittel dient.

*Ausführung der Erfindung*

Zur Herstellung von Isobuttersäure oder ihren Estern wird je Mol eingesetzten Propylens mindestens etwa 1 mol Wasser bzw. 1 mol eines Alkanols eingesetzt. Das bevorzugte Alkanol ist Methanol. Andere Alkanole, die in Betracht kommen, sind Äthanol, Propanol, Isopropanol oder n- oder Isobutanol. Vorzugsweise wird die Gesamtmenge des zur Umsetzung eingesetzten Wassers oder Alkanols erfindungsgemäss als Waschflüssigkeit in dem Absorber eingesetzt, jedoch braucht diese Menge nicht über 2 mol je Mol eingesetzten Propylens gesteigert zu werden.

Die Kochsche Synthese von Isobuttersäure oder ihren Estern wird unter Druck ausgeführt. Übliche Drucke für diese Reaktion sind 50 bis 150 bar. Man kann einen Teil der Gasphase aus dem Druckreaktor abnehmen, unter Aufrechterhaltung des Druckes in einen Absorber leiten und mit dort eingepumptem Wasser oder Alkanol in Berührung bringen. Das hat den Vorteil, dass das korrosive Gemisch aus dem Wasser oder Alkanol und dem Fluorwasserstoff nicht mittels einer Druckpumpe in diesem Falle der Absorber druckfest ausgeführt werden. Am Ausgang des Absorbers wird ein Entspannungsventil angeordnet, mit dem zugleich die Menge des Abgasstromes eingestellt werden kann. Das entspannte Abgas kann zur Entfernung von Restmengen Fluorwasserstoff über einen Absorber mit der bereits erwähnten basischen Füllung geleitet werden. Schliesslich kann das daraus entweichende Gas mit Luft vermischt und entzündet werden, um organische Bestandteile zu verbrennen. Falls es nicht von allein brennt, kann ein brennbares Gas zugemischt werden.

Bei der Entspannung der flüssigen Phase des Reaktionsgemisches zwecks weiterer Aufarbeitung und gegebenenfalls bei der destillativen Zerlegung des flüssigen Reaktionsgemisches werden infolge der Druckentlastung gasförmige Bestandteile freigesetzt, die zuvor gelöst waren, vor allem Fluorwasserstoff. Wenn die auszuschleusenden Fremdgase zu einem erheblichen Anteil bei dieser Gelegenheit freigesetzt werden, genügt es, nur die bei der Entspannung freiwerdenden Gase über den Absorber zu leiten. Man braucht in diesem Falle kein Gas direkt aus dem Druckbereich zu entnehmen. Es kann in mehreren Stufen, z.B. erst auf 2 bis 10 bar, dann auf Atmosphärendruck entspannt werden, wobei jedesmal Gase freigesetzt werden. Es ist im allgemeinen nicht wirtschaftlich, dieses korrosive Gas zu komprimieren, um es in den Reaktionsraum zurückführen zu können. Es wird daher in der Regel in den Absorber geleitet und nach Verbrennung der organischen Bestandteile und Herauswaschen des Fluorwasserstoffs in die Atmosphäre entlassen.

Wenn der durch die Entspannung der Flüssigphase freiwerdende Gasstrom nicht ausreicht, um die anfallenden Fremdgase auszuschleusen, muss ein Teil der Gasphase aus dem Druckbereich abgenommen werden. Sofern es nicht unter Druck ausgewaschen wird, wird es entspannt und zweckmässigerweise mit dem aus der Flüssigphase austretenden Gas vereinigt. Beim Auswaschen des entspannten Gases wird Fluorwasserstoff enthaltendes Wasser oder Alkanol gewonnen, das mit einer geeigneten Flüssigkeitspumpe in den Druckbereich gefördert wird.

Zusätzliche Massnahmen können erforderlich werden, wenn das ausgeschleuste Gas nennenswerte Anteile an Isopropylfluorid enthält. Dieses gasförmige Produkt wird im Absorber von dem Wasser oder Alkanol nicht aufgenommen. Um zu verhindern, dass es in die Atmosphäre entweicht, wird dem Gas Sauerstoff, vorzugsweise in Form von Luft, beigemischt und die organischen Bestandteile verbrannt. Falls es von allein nicht brennt, wird ein brennbares Material zugemischt. Dabei entsteht aus dem Isopropylfluorid neben Kohlendioxid und Wasser gasförmiger Fluorwasserstoff. Das Verbrennungsgas wird in den Absorber geleitet, wo auch der bei der Verbrennung entstandene Fluorwasserstoff ausgewaschen wird.

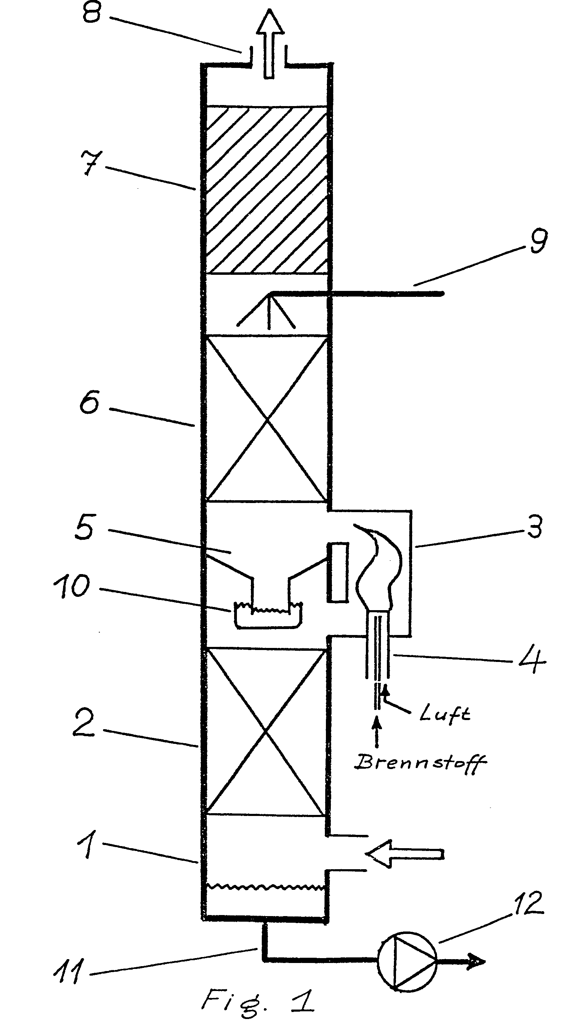

Bei Anwendung dieser zusätzlichen Verbrennungsstufe ist es vorteilhaft, den Absorber in zwei Stufen zu unterteilen und die Verbrennungsstufe dazwischen anzuordnen. Eine dafür geeignete Vorrichtung ist in Fig. 1 im Querschnitt dargestellt. Der Gasstrom tritt in die Vorkammer 1 der ersten Absorberstufe 2 ein. Nach Durchlauf durch den Absorber 2 gelangt es in die Brennkammer 3, in die über eine Leitung 4 Sauerstoff oder Luft und gegebenenfalls ein brennbares Material eingeführt werden. Das Gasgemisch wird verbrannt und gelangt nach der Verbrennung in die Vorkammer 5 der zweiten Absorberstufe 6, aus der es in eine dritte Absorberstufe 7 gelangt, wo Restmengen des Fluorwasserstoffs an feste oder flüssige Absorptionsmittel gebunden werden. Danach tritt das Gas über die Leitung 8 in die Atmosphäre aus. Das als Waschflüssigkeit eingesetzte Wasser oder Alkanol gelangt durch die Leitung 9 auf die zweite Absorberstufe 6 und tritt aus der Vorkammer 5 über den Überlauf 10 in die untere Absorberstufe 2 ein. Es sammelt sich in der Vorkammer 1 und wird von dort über die Leitung 11 und erforderlichenfalls eine Druckpumpe 12 in den Reaktionsraum eingeleitet.

Wenn beide Absorberstufen 2 und 6 unter dem gleichen Druck betrieben werden, genügt ein dazwischen angeordneter Überlauf 10, der den direkten Durchtritt des Gasstromes verhindert und diesen zum Durchgang durch die Brennkammer 3 zwingt. Gegebenenfalls kann die erste Absorberstufe 2 unter Druck betrieben werden. Dann wird in dem Übergang zur Brennkammer 3 ein Entspannungsventil angeordnet und die Waschflüssigkeit wird aus der Vorkammer 5 mittels einer Druckpumpe auf den Kopf des Absorbers 2 befördert.

Die Absorber 2 und 6 enthalten im Regelfall übliche Füllkörper. Diese, ebenso wie alle übrigen Teile der Absorptionsanlage, sind aus einem gegenüber Fluorwasserstoff bzw. wässeriger Flusssäure beständigen Werkstoff gefertigt. Geeignet sind z.B. Aluminium, Nickel-Chrom-Eisen-Legierungen oder Polytetrafluoräthylen oder ähnliche fluorierte Polymere.

Die Menge des ausgeschleusten Gases wird so gross bemessen, dass der darin enthaltene Anteil an Fremdgasen und unerwünschten gasförmigen Nebenprodukten dem laufenden Zustrom und der Neubildung entspricht. Da mit den Fremdgasen stets ein Teil des Propylens und Kohlenmonoxids verloren geht, ist es vorteilhaft, wenn der Partialdruck der Fremdgase hoch liegt. Man kann daher den Partialdruck der Fremdgase in der Gasphase des Reaktionsraumes so stark anwachsen lassen, dass eine spürbare Beeinträchtigung des Reaktionsablaufes gerade noch vermieden wird. Im laufenden Betrieb stellt sich ein gleichbleibender Partialdruck der Fremdgase ein. Dieser Partialdruck soll 30 bar möglichst nicht überschreiten. Der Gesamtdruck muss so hoch gehalten werden, dass nach Abzug des Partialdruckes der Fremdgase der Druck der Reaktionsgase, nämlich Propylen, Kohlenmonoxid und Fluorwasserstoff, hoch genug ist, um die erwünschte Reaktionsgeschwindigkeit zu gewährleisten.

**Patentansprüche**

1. Verfahren zur kontinuierlichen Herstellung von Isobuttersäure oder ihren Alkylestern durch Kochsche Synthese aus Propylen, Kohlenmonoxid und Wasser oder einem niederen Alkanol in Gegenwart von Fluorwasserstoff als Kochschem Katalysator in wenigstens einem Reaktionsraum, der eine flüssige und eine gasförmige Phase unter hohem Druck enthält, und anschliessende, wenigstens teilweise Entspannung in eine Aufarbeitungsvorrichtung, dadurch gekennzeichnet, dass wenigstens ein Teil des beim Entspannen aus der flüssigen Phase austretenden Gases und gegebenenfalls ein Teil der gasförmigen Phase aus dem Reaktionsraum aus der Aufarbeitungsvorrichtung ausgeschleust und in einem Absorber mit wenigstens einem Teil des zur Kochschen Synthese eingesetzten Wassers oder niederen Alkanols in Berührung gebracht und das aus dem Absorber abfliessende Wasser bzw. Alkanol in den Reaktionsraum eingeleitet wird.

2. Verfahren nach Anspruch 1, dadurch gekennzeichnet, dass dem ausgeschleusten Gas Sauerstoff zugesetzt wird und die darin enthaltenen organischen Verbindungen wenigstens teilweise verbrannt werden und dass das Gas danach in dem Absorber mit dem Wasser oder Alkanol in Berührung gebracht wird.

3. Verfahren nach einem der Ansprüche 1 oder 2, dadurch gekennzeichnet, dass das aus dem Absorber austretende Gas mit einem basischen Absorptionsmittel in Berührung gebracht wird.

**Claims**

1. Process for continously producing isobutyric acid or the alkyl esters thereof by Koch's synthesis from propylene, carbon monoxide and water or a lower alkanol in the presence of hydrogen fluoride as Koch's catalyst in at least one reaction chamber which contains a liquid and a gaseous phase under high pressure and subsequently at least partially releasing the pressure in a processing apparatus, characterised in that at least some of the gas which comes out of the liquid phase when the pressure is released and possibly some of the gaseous phase from the reaction chamber is flushed out of the processing apparatus and contacted with at least some of the water or lower alkanol used for the Koch's synthesis in an absorber and the water or alkanol flowing out of the absorber is passed into the reaction chamber.

2. Process as claimed in Claim 1, characterised in that oxygen is added to the gas flushed out and the organic compounds contained therein are at least partially burnt up and then the gas is contacted in the absorber with the water or alkanol.

3. Process as claimed in Claim 1 or 2, characterised in that the gas leaving the absorber is contacted with a basic absorption agent.

**Revendications**

1. Procédé pour la production en continu d'acide isobutyrique ou de ses esters alcoyliques par synthèse de Koch à partir de propylène, d'oxyde de carbone et d'eau ou d'un alcanol inférieur en présence de fluorure d'hydrogène servant de catalyseur de Koch, dans au moins une zone de réaction qui contient une phase liquide et une phase gazeuse sous haute pression, suivie d'une détente, au moins partielle, dans un dispositif de traitement ultérieur, caractérisé en ce qu'une partie au moins du gaz qui s'échappe de la phase liquide lors de la détente et, le cas échéant, une partie de la phase gazeuse sont éclusées hors de la zone de réaction vers le dispositif de traitement ultérieur et mises en contact, dans un absorbeur, avec une partie au moins de l'eau ou de l'alcanol inférieur utilisé pour la synthèse de Koch, et en ce que l'eau ou l'alcanol qui sort de l'absorbeur est envoyé dans la zone de réaction.

2. Procédé selon la revendication 1, caractérisé en ce que de l'oxygène est ajouté au gaz éclusé, en ce que les composés organiques contenus dans celui-ci sont brûlés au moins partiellement et en ce que le gaz est mis ensuite en contact, dans l'absorbeur, avec l'eau ou l'alcanol.

3. Procédé selon l'une des revendications 1 ou 2, caractérisé en ce que le gaz qui sort de l'absorbeur est mis en contact avec un produit absorbant basique.

0 079 497

Fig. 1

Brennstoff

Luft